# EUROPÄISCHE PATENTSCHRIFT

(11) **EP 1 895 926 B1**
(45) Veröffentlichungstag und Bekanntmachung des Hinweises auf die Patenterteilung: **04.12.2013**
(21) Anmeldenummer: 06777479.4
(22) Anmeldetag: 27.06.2006
(51) Int. Cl.: A61B 18/14, A61B 17/32, A61B 17/3205

(54) **POLYPEKTOMIESCHLINGE**
POLYPECTOMY SNARE
BOUCLE DE POLYPECTOMIE

(30) Priorität: 29.06.2005 DE 102005030159
(43) Veröffentlichungstag der Anmeldung: 12.03.2008
(73) Patentinhaber: MTW-Endoskopie W. Haag KG, 46487 Wesel (DE)
(72) Erfinder: HAAG, Wolfgang, 46487 Wesel (DE)
(74) Vertreter: Rieder, Hans-Joachim
(86) Internationale Anmeldenummer: PCT/EP2006/063585
(87) Internationale Veröffentlichungsnummer: WO 2007/000452

(56) Entgegenhaltungen:
- DE-A1- 19 953 359
- US-A- 5 437 665
- US-A1- 2003 109 874
- US-A1- 2003 139 750

## Beschreibung

Die Erfindung betrifft eine Polypektomieschlinge mit einem in einer Hülse geführten Zug-/Schubelement zum Übertragen einer Zug-/Schubkraft von einer an dem einen Ende der Hülse angeordneten Betätigungseinrichtung auf eine an dem anderen Ende der Hülse angeordnete Schlinge, um die Schlinge von einer Verwahrstellung, in welcher die Schlinge in einer Gestrecktlage in der Hülse einliegt, in eine Gebrauchsstellung, in welcher die aufgespreizte Öffnung der Schlinge vor der Hülse liegt, und wieder zurück zu verlagern. Die Polypektomieschlinge weist insbesondere einen zusammen mit der Schlinge verlagerbaren, gleitend in der Hülse gelagerten Anschlag auf, der beim Verlagern der Schlinge von ihrer Verwahrstellung in ihre Gebrauchsstellung gegen einen hülsenfesten Gegenanschlag tritt, um eine Spreizwirkung auf die Schlinge auszuüben.

Eine derartige Polypektomieschlinge ist aus dem Gebrauchsmuster DE 7835595 vorbekannt. Die vorbekannte Polypektomie- oder Diatermieschling besteht aus einem Zugseil, das am vorderen Ende in eine Schlinge von einer halkreisähnlichen Form ausläuft. Die Schlinge weist eine Spitze auf. Das Zugseil ist innerhalb der Hülse und einem am Hülsenende angeordneten rohförmigen Gegenanschlagteil leicht verschiebbar geführt. Ein Ende der beiden Schlingenschenkel ist fest mit dem Zugseil verbunden, so dass mit dem Zugseil die Schlinge aus einer Verwahrstellung, in welcher sie in einer Gestrecktlage der Hülse einliegt, in eine Gebrauchsstellung verlagerbar ist, in welcher die Schlinge vor dem Ende der Hülse liegt. Der andere Schlingenchenkel ist mit seinem in der Hülse liegenden Ende mit einem Anschlag verbunden, der im Zuge des Herausschiebens der Schlinge aus der Hülse gegen den Gegenanschlag tritt, so dass sich die Schlinge halbkreisförmig aufspreizt, wenn der andere Schlingenschenkel weitergeschoben wird.

Aus US 5,437,665 A ist eine Polypektomieschlinge mit einer von der Federkraft der V-förmig abgespreizten Spreizschenkel induzierten Aufspreizung der Schlinge bekannt.

Aus der DE 2951060 ist eine hohe Frequenzresektionsvorrichtung bekannt, mit welcher eine Schlinge der vorbeschriebenen Art bestromt werden kann. Die Schlinge wirkt dann als Elektrode. Die so ausgebildete Vorrichtung dient der Resektion abdominalen Gewebes, wie zum Beispiel einer Geschwulst, mittels hochfrequenter Ströme nach einem System zur Resektion eines in einer Körperhöhle, insbesondere im Darm befindlichen und eine Geschwulst, beispielsweise einen oder mehrere Polypen aufweisenden Gebiets, bei dem man einen Hochfrequenzstrom zwischen einer zur Resektion bestimmten Elektrode, die von dem in die Höhlung eingeführten schlingenförmigen leitenden Draht-gebildet wird, und einer äußeren Elektrode fließen lässt, die fest auf der Haut eines Patienten aufliegt und von der Resektionselektrode getrennt angeordnet ist.

Der Chirurg muss dabei die Schlinge, die üblicherweise mittels eines Endoskops in die Körperhöhlung eingeführt wird, über den Polypen legen.

Der Erfindung liegt die Aufgabe zugrunde, die Handhabbarkeit des gattungsgemäßen chirurgischen Schneidgerätes zu verbessern.

Gelöst wird die Aufgabe durch die in den Ansprüchen angegebene Erfindung. Jeder der Ansprüche stellt grundsätzlich eine eigenständige Lösung der der Erfindung zugrunde liegenden Aufgabe dar und ist mit jedem anderen Anspruch in beliebiger Form kombinierbar.

In der Erfindung, die in Anspruch 1 definiert wird, erstrecken sich parallel zu den Spreizelementen Zugelemente. Diese Zugelemente können von Seilen ausgebildet sein, die mit ihrem einen Ende jeweils am freien Ende des Spreizschenkels angreifen. Sie sind dort fest mit dem Spreizschenkel verbunden. Das andere Ende des jeweiligen Zugelementes durchragt die Öffnung der Hülse und insbesondere einen in dem bereich des Endes angeordneten Gegenanschlag in Form eines Rohrabschnittes. Innerhalb der Hülse sind die beiden Enden der Zugelemente mit einem Anschlag verbunden. Dieser Anschlag ist zusammen mit der Schlinge verlagerbar. Er ist aber der Schlinge gleitend zugeordnet. Der Anschlag besitzt ebenfalls eine Rohrform. Durch die Rohröffnung ragen die Spreizschenkel. Da die von Seilen ausgebildeten Zugelemente vorzugsweise jeweils an den Außenbreitseiten der Spreizschenkel entlang laufen, üben sie einen Zug auf die freien Enden der Spreizschenkel aus, wenn der Anschlag im Zuge des Verlagerns der Schlinge von der Verwahrstellung in die Gebrauchsstellung gegen den Gegenanschlag tritt. Dann werden nämlich die Zugelemente gespannt und bewirken eine Verstärkung des Aufspreizens der Schlinge. Dies erfolgt in der Endphase der Verlagerung der Schlinge von der Verwahrstellung in die Gebrauchsstellung. Durch die Zugelemente ist nach dem Inanschlagtreten des Anschlages gegen den Gegenanschlag die Abstandsstrecke des freien Endes des Spreizschenkels vom Ende der Hülse festgelegt. Werden dann die Spreizschenkel weiter aus der Hülse durch einen vom Betätigungselement ausgeübten Druck weiter herausgeschoben, so müssen sich die Spreizschenkel verbiegen. Die beiden freien Enden der Spreizelemente treten dabei weiter voneinander weg. Dies unterstützt eine Knickstelle etwa in der Mitte jedes Spreizschenkels.

Ein weiteres Ausführungsbeispiel sieht vor, dass die Schlinge zwei in der Gebrauchsstellung teilweise in und vor der Hülse liegende V-förmig abgespreizte Spreizschenkel ausbildet, die zumindest in Richtung quer zur Öffnungsebene biegesteifer sind als ein die jeweiligen freien Enden der Spreizschenkel miteinander verbindender Schlingenbogen. Zufolge dieser Ausgestaltung kann die Schlinge mit ihrer Spitze gegen die Wandung der Körperhöhle gedrückt werden. Dabei biegt sich der weniger biegesteife Schlingenbogen ab, so dass der Bereich in Blickrichtung vom Ende der Hülse auf den Scheitel der Schlinge unmittelbar nach den freien Enden der Speizschenkel ein Bereich größter Durchbiegung ist. Dieser Bereich ist gleichzeitig der Bereich der größten Öffnungsweite der Schlinge, so dass die im besten Falle rechtwinklig aufgebogene, aufgespreizte Schlinge über den Polypen gehoben bzw gezogen werden kann. Die Spreizschenkel können von streifenartigen, federelastischen Elementen gebildet werden. Vorzugsweise handelt es sich um blattfederartige Metallstreifen, die mit ihren Breitseiten in der Verwahrstellung gegeneinander liegen. Die Spreizschenkel sind vorgespannt, so dass sie aus der Parallellage, in der sie in der Verwahrstellung liegen beim Durchtritt durch die Öffnung der Hülse ihre V-förmige Spreizstellung einnehmen. Der die freien Enden der Spreizschenkel miteinander verbindende Schlingenbogen kann aus einem Drahtseil oder dergleichen ausgebildet sein, welches weniger biegesteif ist als die Spreizschenkel. Der Schlingenbogen kann eine deutliche ausgeprägte Spitze mit spitzwinklig zueinander verlaufenden Abschnitten aufweisen. Im Spitzenbereich können die Abschnitte des Schlingenbogens sogar parallel zueinander verlaufen, so dass der Scheitel des Schlingenbogens eine Art Bucht ausbildet. Des Weiteren ist vorgesehen, das zumindest der Schlingenbogen mit einer Vielzahl von lokalen Materialverstärkungen, insbesondere Verdickungen versehen ist. Dies dient dem besseren Anhaften des, durch Einleiten des oben erwähnten Stromes von dem Gewebe der Körperhöhlung abgetrennten Polypen an der Schlinge. Es ist alternativ vorgesehen, dass der Schlingenbogen eine Vielzahl von schlingeneinwärts gerichteten Vorsprüngen aufweist, die Haken ausbilden, um ebenfalls den abgetrennten Polypen an der Schlinge zu halten, damit er aus der Körperhöhlung heraustransportiert werden kann.

Ausführungsbeispiele der Erfindung werden nachfolgend anhand beigefügter Zeichnungen erläutert. Es zeigen
- Fig. 1a: das erste, die Schlinge aufweisende Ende eines ersten Ausführungsbeispiels in vergrößerter Darstellung;
- Fig. 1b: das andere Ende dieses Ausführungsbeispiels mit Betätigungseinrichtung;
- Fig. 2a: eine Darstellung gemäß Fig. 1a, wobei die Schlinge in die Gebrauchsstellung verlagert ist;
- Fig. 2b: eine Darstellung gemäß Fig. 1b in der Gebrauchsstellung;
- Fig. 3a: eine Darstellung gemäß Fig. 2a, wobei die Schlinge in die Aufspreizstellung verlagert ist;
- Fig. 3b: eine Darstellung gemäß Fig. 2b in der Aufspreizstellung;

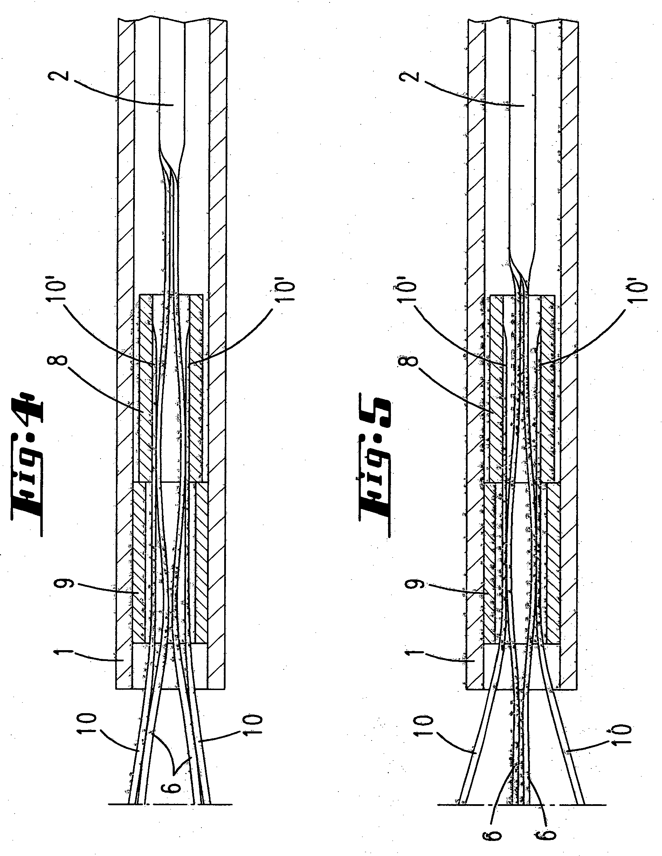

- Fig. 4: den in Fig. 2a mit IV - IV bezeichneten Bereich in vergrößerter Darstellung;
- Fig. 5: den in Fig. 3a mit V - V bezeichneten Bereich in vergrößerter Darstellung;
- Fig. 6: eine perspektivische Darstellung einer gemäß Fig. 3 aufgespreizten Schlinge, bei der zufolge eines senkrechten Drucks der Schlinge auf eine Unterlage der Schlingenscheitelbogenabscichtt etwa rechtwinklig zur Hülse abgespreizt ist, wobei sich eine Öffnung der Weite Deinstellt;
- Fig. 7: die Schlinge eines zweiten Ausführungsbeispiels in der Aufspreizstellung etwa wie Fig. 3a;
- Fig. 8: die Schlinge eines dritten Ausführungsbeispiels in einer Darstellung wie Fig. 7.

Die Elemente des chirurgischen Schneidgeräts der Ausführungsbeispiele bestehen aus Materialien, die in der Lage sind, den beim Sterilisieren des Gerätes auftretenden Temperaturen stand zur halten. Die Hülse 1 besteht aus einem Kunststoff, beispielsweise PTFT. Die Hülse 1 ist über Formschlusselemente, im Wesentlichen über Steckverbindungen mit einer Betätigungseinrichtung 3 verbunden. Diese besitzt eine Führungsschiene 16, die an ihrem Ende ein Durchgriffsauge für einen Finger, insbesondere den Daumen des Operateurs besitzt. Auf der Führungsschiene 16, die schub- und zugfest mit der Hülse 1 verbunden ist, sitzt ein Zug-Schub-Stück 17, welches zwei sich gegenüberliegende Griffmulden aufweist, um das Zug-Schub-Stück 17 zwischen zwei Fingern des Operateurs halten zu können.

Innerhalb der Hülse 1 befindet sich ein elektrisch leitendes Zug-Schubelement, das im Ausführungsbeispiel als Metallseele, insbesondere in Form eines Seiles oder eines Flachstreifens ausgebildet ist. Wird das Zug-Schub-Stück 17 verlagert, so verschiebt sich das Zug-Schub-Element 2 innerhalb der Hülse 1. Das Zug-Schub-Stück 17 trägt ferner einen elektrischen Kontakt 14, welcher an eine Klemme eines Hochfrequenzstromgenerators angeschlossen werden kann, mit welchem die mit der Bezugsziffer 4 bezeichnete Schlinge des Schneidgeräts bestromt werden kann, um in der bekannten Weise Polypen von dem sie tragenden Gewebe mittels eines Koagulationsstromes abtrennen zu können.

Die Betätigungseinrichtung 3 weist darüber hinaus noch eine Spülöffnung 15 auf, mittels der eine Spülflüssigkeit oder ein Gas in die Hülse 1 eingeleitet werden kann.

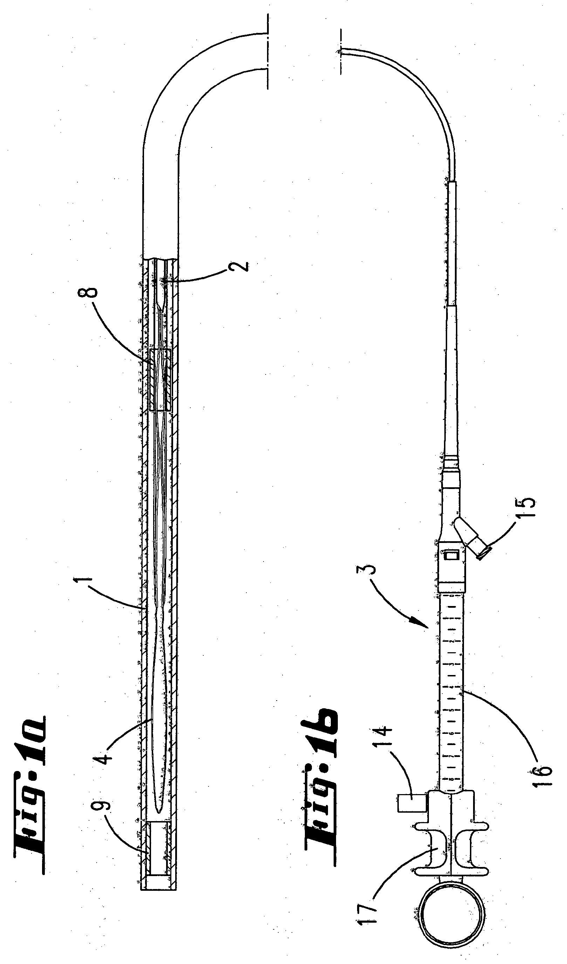

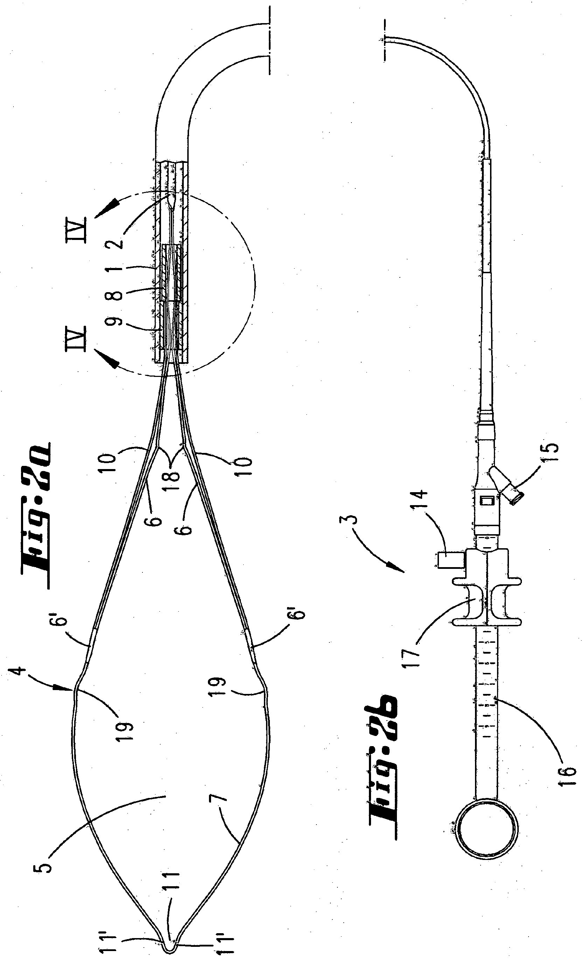

Das Ende des Zug-Schubelementes 2 geht in zwei sich in der Verwahrstellung, in welcher die Schlinge 4 in der Hülse 1 einliegt, im wesentlichen parallel zueinander erstreckende Spreizschenkel 6 über. Bei den Spreizschenkeln 6 handelt es sich jeweils um flache, federelastische Metallstreifen, die mit ihren Breitseitenflächen aneinander liegen. Die beiden Spreizschenkel 6 sind federvorgespannt und besitzen eine Knickstelle 18, so dass sie beim Herausfahren aus der Hülse 1 V-förmig abspreizen, wie es die Figur 2a zeigt. Bei der Verlagerung der Schlinge 4 aus der Hülse 1 von der in Figur 1a dargestellten Verwahrstellung, in welcher die Schlinge 4 in einer Gestrecktlage vollständig in der Hülse einliegt, bis in die in Figur 2a dargestellte Gebrauchsstellung, in welcher die Knickstellen 18 außerhalb vor dem Ende der Hülse 1 liegen, spreizer sich die beiden Spreizschenkel 6 V-förmig auf, so dass der Schlingenscheitelbogen 7, mit dem die freien Enden 6' der Spreizschenkel 6 miteinander verbunden sind, sich aufspreizt. Der Zwischenraum zwischen den aufgespreizten Spreizschenkeln 6 bildet dabei einen Bereich der Schlingenöffnung 5. Die Schlingenöffnung 5 wird durch den Schlingenscheitelbogen 7 geschlossen, der aus einem Drahtseil besteht und weniger biegesteif ist als die beiden Spreizschenkel 6.

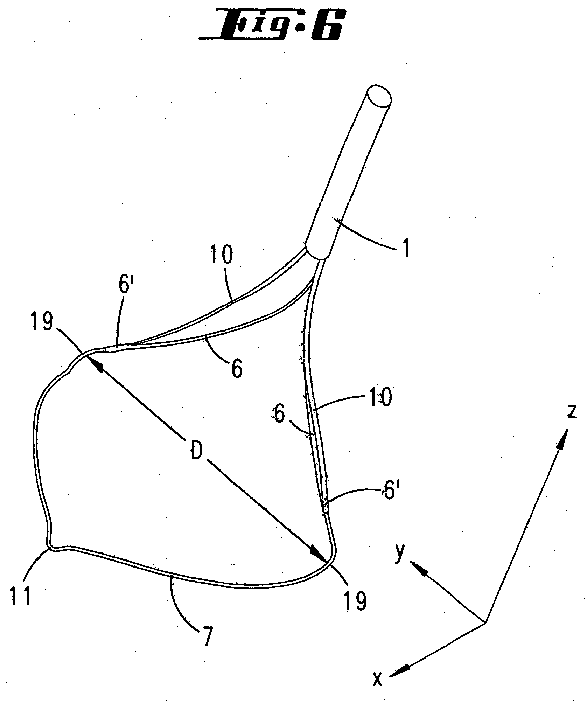

Der Scheitel 11 des Schlingenscheitelbogens 7 bildet eine Verengung in Form einer Bucht aus, wobei die beiden Abschnitte 11' dieser Bucht spitzwinklig zueinander verlaufen. Auch im Bereich des Schlingenscheitelbogens 7, der benachbart zum freien Ende 6' des Spreizschenkels 6 liegt, bildet eine Ausbuchtung 19 nach außen aus. Wie der Figur 6 zu entnehmen ist, liegen sich die deiden Ausbuchtungen 19 im wesentlichen diametral gegenüber und bilden den Bereich des größten Durchmessers D der Schlinge.

An den freien Enden 6' der Spreizschenkel 6 sind jeweils Zugelemente 10 befestigt. Diese Zugelemente 10 bestehen aus dünnen Drahtseilen und durchragen zusammen mit den Spreizschenkeln 6 einen rohrförmigen Gegenanschlag 9, der innerhalb der Hülse 1 an deren Ende schubfest angeordnet ist. Die Zugelemente 10 sind mit ihren anderen Enden an einem Anschlagrohrstück 8 befestig. Dieses Anschlagrohrstück ist gleitend auf den in der Hülse 1 liegenden Abschnitten der Spreizschenkel 6 gelagert. Beim Verlagern der Schlinge 4 zwischen der Verwahrstellung und einer ersten Öffnungsstellung gleitet dieses Anschlagrohrstück 8 innerhalb der Hülse 1. Es besitzt hierzu einen geringeren Durchmesser als das Gegenanschlagrohrstück 9. Die Zugelemente 10 verlaufen außerhalb der Schlingenöffnung 5 im wesentlichen parallel zu den Spreizschenkeln 6. Zufolge der Knickstellen 18 liegen die Zugelemente 10 jedoch geringfügig beabstandet zu den Spreizschenkel 6.

Wird die Schlinge 4 von der in Figur 1 dargestellten Verwahrstellung in die in Figur 2a dargestellte erste Öffnungsstellung verlagert, so spreizen sich die Spreizschenkel 6 zufolge ihrer Federvorspannung voneinander V-förmig ab. In dieser Stellung liegt das Anschlagrohrstück 8 am Gegenanschlagrohrstück 9 an.

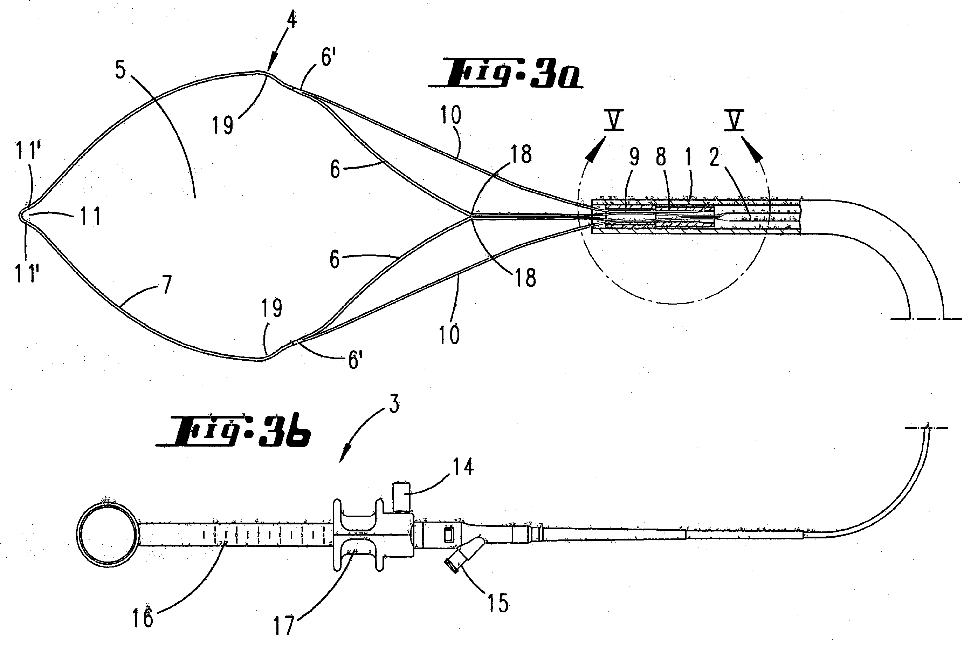

Durch Aufbringen eines weiteren Schubs mittels des Zug-Schub-Stücks 17 auf das Zug-Schubelement 2 werden die Spreizschenkel 6-geringfügig weiter aus der Hülse 1 heraus verlagert. Da sich die Zugelemente 10 zufolge des gegen den Gegenanschlag 9 getretenen Anschlags 8 nicht weiter aus der Hülse 1 verlagern können, führt dies zu einer Durchbiegung der Spreizschenkel 6, was dazu führt, dass sich die Knickstellen 18 weiter von den Zugelementen 10 entfernen und sich aufeinander zu bewegen, wie dies in der Figur 3a dargestellt ist. Dabei entfernen sich die freien Enden 6' voneinander und die Schlinge 4 spreizt sich weiter auf. Wenn dabei die Knickstellen 18 gegeneinander stoßen, wird dieses Aufspreizen sogar noch verstärkt.

Wird die Schlinge 4 senkrecht gegen einen Untergrund gedrückt, so verbiegt sich der Schlingenscheitelbogen 7, wie es in der Figur 6 dargestellt ist. Dort ist der Schlingenscheitelbogen 7 im wesentlichen rechtwinklg zur Ebene, welche von den V-förmigen Spreizschenkeln 6 aufgespannt wird, rechtwinklig abgebogen. Dies ist eine Folge der stärkeren Biegesteifigkeit der Spreizschenkel 6 insbesondere in Richtung quer zur Schlingenöffnung 5, was eine Folge der Orientierung der schlanken Blattfedern ist, von denen die Spreizschenkel 6 ausgebildet werden. Zur Verdeutlichung sind in der Figur 6 die drei senkrecht aufeinander stehenden Raumrichtungen mit X, Y, Z bezeichnet. Die Hülse 1 erstreckt sich dort parallel zur Z-Richtung. Der Schlingenschetelbogen 7 ist in eine Ebene abgebogen, die der X-Y-Ebene entspricht. Wie der Figur 6 zu entnehmen ist, biegt sich die Schlinge 4 im Bereich der Ausbuchtungen 19 durch, also in einem Bereich, der unmittelbar den freien Enden 6' der Spreizschenkel 6 benachbart ist. Dies ist der Bereich mit der größten Öffnungsweite der Schlinge 4, die bei der Verwendung des Gerätes als chirurgisches Schneidgerät über einen Polypen oder dergleichen gelegt werden kann.

Ist die Schlinge 4 über einen Polypen gelegt, so wird auf das Zug-Schub-Stück 17 ein Zug ausgeübt, so dass sich die Schlinge zusammenzieht, um den Polypen insbesondere an seinem Hals eng zu umfassen. Dann erfolgt in der bekannten Weise das Abtrennen des Polyps durch den über die Elektrode 14 eingeleiteten Strom.

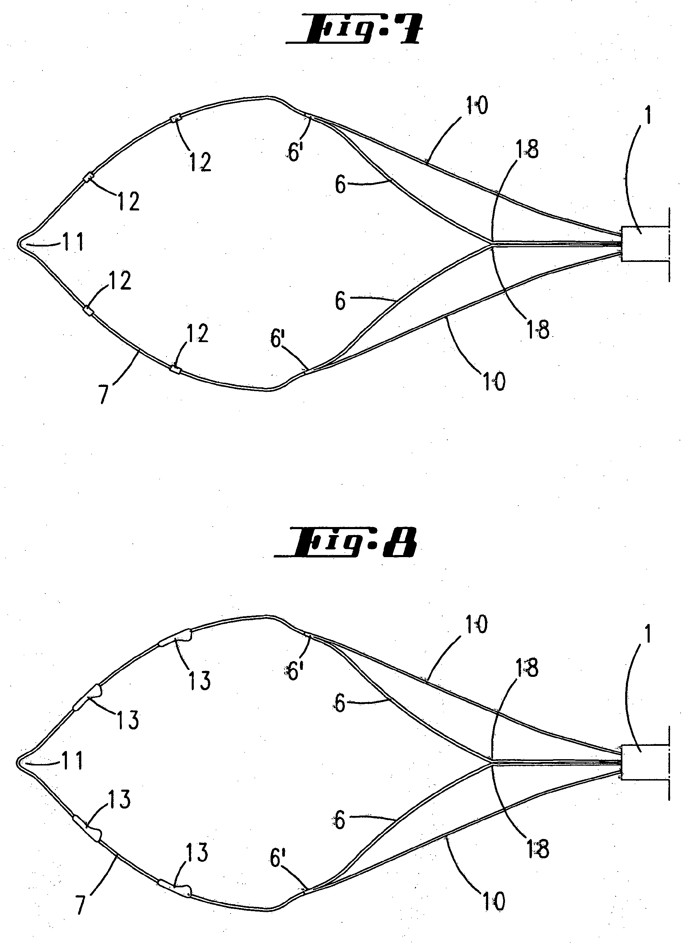

Um den abgetrennten Polypen besser inner Schlinge 4 halten zu können, sind bei dem in der Figur 7 dargestellten zweiten Ausführungsbeispiel Verdickungen 12 im Bereich des Schlingenscheitelbogens 7 vorgesehen

Bei dem in der Figur 8 dargestellten Ausführungsbeispiel sind insgesamt vier schlingeneinwärts gerichtete Haken 13 vorgesehen, die der Halterung des abgetrennten Polypens in der Schlinge 4 dienen.

Hervorzuheben ist, dass die Querschnittsgestalt der Spreizschenkeln 6 so gewählt ist, dass sie sich innerhalb der Ebene der Schlingenöffnung 5 leichter verbiegen als in Querrichtung dazu. Hierzu besitzen die Spreizschenkel 6 bevorzugt einen rechteckigen Querschnitt, wobei die Rechteckkanten, die die Breitseitenflächen der Spreizschenkel 6 bilden, um ein Vielfaches länder sind als die rechtwinklig dazu stehenden Schmalkanten, die in der Ebene der Schlingenöffnung 5 liegen. Nicht nur in der Verwahrstellung, sondern auch in der Gebrauchsstellung weisen die Breitseitenflächen der Spreizschenkel 6 aufeinander zu. Sie liegen in der Verwahrstellung zumindest bereichsweise in berührender Anlage.

## Patentansprüche

1. Chirurgisches Schneidgerät mit einem in einer Hülse (1) geführten Zug-/Schubelement (2) zum Übertragen einer Zug-/Schubkraft von einer an dem einen Ende der Hülse (1) angeordneten Betätigungseinrichtung (3) auf eine an dem anderen Ende der Hülse (1) angeordnete Schlinge (4), um die Schlinge (4) von einer Verwalustellung, in welcher die Schlinge (4) in einer Gestrecktlage in der Hülse (1) einliegt, in eine Gebrauchsstellung, in welcher die aufgespreizte Öffnung (5) der Schlinge (4) vor der Hülse (1) liegt, und wieder zurück zu verlagern, wobei die Schlinge zwei in der Gebrauchsstellung teilweise in und vor der Hülse (1) liegende V-förmig abgespreizte Spreizschenkel (6) ausbildet, deren freien Enden (6') mit einem Schlingenbogen (7) verbunden sind, **dadurch gekennzeichnet, dass** ein zusammen mit der Schlinge (4) verlagerbarer, gleitend in der Hülse (1) gelagerter Anschlag (8) beim Verlagern der Schlinge (4) von ihrer Verwahrstellung in ihre Gebrauchsstellung gegen einen hülsenfesten Gegenanschlag (9) tritt, um eine Spreizwirkung auf die Schlinge (4) auszuüben, und dass an jedem der freien Enden (6') zusätzliche, im wesentlichen parallel zu den Spreizschenkeln (6) sich erstreckende Zugelemente (10) angreifen, die mit dem gegenüber den Spreizschenkeln (6) verschieblichen Anschlag (8) verbunden sind.

2. Chirurgisches Schneidgerät nach Anspruch 1, **dadurch gekennzeichnet, dass** die V-förmig abgespreizten Spreizschenkel (6) zumindest in Richtung quer zur Öffnungsebene (5) biegesteifer sind als der Schlingenbogen (7).

3. Chirurgisches Schneidgerät nach einem oder mehreren der vorangehenden Ansprüche, **dadurch gekennzeichnet, dass** die Spreizschenkel (6) von federelastischen flachen Streifen ausgebildet sind, die in der Verwahrstellung mit ihren Breitseitenflächen aneinander liegen.

4. Chirurgisches Schneidgerät nach einem oder mehreren der vorangehenden Ansprüche, **dadurch gekennzeichnet, dass** der Schlingenbogen (7) von einem Drahtseil ausgebildet ist.

5. Chirurgisches Schneidgerät nach einem oder mehreren der vorangehenden Ansprüche, **dadurch gekennzeichnet, dass** der Schlingenbogen (7) im Bereich seiner Spitze eine Bucht (11) spitzwinklig oder parallel zueinander verlaufende Schlingenbogenabschnitte (11') ausbildet.

6. Chirurgisches Schneidgerät nach einem oder mehreren der vorangehenden Ansprüche, **dadurch gekennzeichnet, dass** die an den freien Enden (6') der Spreizschenkel (6) angreifenden Zugelemente (10) von Drahtseilen ausgebildet sind.

7. Chirurgisches Schneidgerät nach einem oder mehreren der vorangehenden Ansprüche, **dadurch gekennzeichnet, dass** der Anschlag (8) von einem in der Hülse (1) gleitend gelagerten Rohrstück ausgebildet ist, durch welches Rohrstück die Spreizschenkel (6) hindurchragen.

8. Chirurgisches Schneidgerät nach einem oder mehreren der vorangehenden Ansprüche, **dadurch gekennzeichnet, dass** die Zugelemente (10) sich entlang der voneinander weg weisenden Breitseitenflächen der Spreizschenkel (6) erstrecken,

9. Chirurgisches Schneidgerät nach einem oder mehreren der vorangehenden Ansprüche, **dadurch gekennzeichnet, dass** die freien Enden (6') der Spreizschenkel (6) in Richtung auf den Bogenscheitel der Schlinge (4) vor dem Bereich der größten Öffnungsweite der Schlinge (4) liegen.

10. Chirurgisches Schneidgerät nach einem oder mehreren der vorangehenden Ansprüche, **dadurch gekennzeichnet, dass** zumindest der Schlingenbogen (7) eine Vielzahl von voneinander beabstandeten lokalen Verdickungen (12) aufweist.

11. Chirurgisches Schneid gerät nach einern oder mehreren der vorangehenden Ansprüche, **dadurch gekennzeichnet, dass** zumindest der Schlingenbogen (7) eine Vielzahl von voneinander beabstandeten, schlingeneinwärts gerichteten Haken (13) aufweist.

12. Chirurgisches Schneidgerät nach einem oder mehreren der vorangehenden Ansprüche, **dadurch gekennzeichnet dass** die Schlinge (4) aus einem elektrisch leitenden Material besteht und über das als Band oder Seil ausgebildete Zug-/Schubelement (2) mit einem elektrischen Kontakt (14) der Betätigungseinrichtung elektrisch leitverbunden ist.

## Claims

1. Surgical cutting device comprising a traction/thrust element (2) guided in a sleeve (1) for transferring a tractive/thrust force from an actuation means (3) arranged at one end of the sleeve (1) to a loop (4) arranged at the other end of the sleeve (1) in order to displace the loop (4) from a storage position, in which the loop (4) is enclosed in an elongated position in the sleeve (1), into a usage position, in which the expanded opening (5) of the loop (4) is located in front of the sleeve (1), and back again, the loop forming two V-shaped splayed expansion legs (6) which are located in part inside and in front of the sleeve (1) in the usage position, the free ends (6') of which legs are connected to a loop arc (7), **characterised in that** when the loop (4) is displaced, a stop (8) which can be displaced together with the loop (4) and is slidably mounted in the sleeve (1) is displaced from the storage position thereof into the usage position thereof against a counterstop (9) which is fixed in the sleeve, in order to exert an expansion effect on the loop (4), and **in that** additional traction elements (10) which extend substantially parallel to the expansion legs (6) engage with each of the free ends (6'), which traction elements are connected to the stop (8) which can be moved relative to the expansion legs (6).

2. Surgical cutting device according to claim 1, **characterised in that** the V-shaped splayed expansion legs (6) are more rigid at least in the direction transverse to the opening plane (5) than the loop arc (7).

3. Surgical cutting device according to one or more of the preceding claims, **characterised in that** the expansion legs (6) are formed of resilient planar strips which are adjacent to each other at the wide side faces thereof in the storage position.

4. Surgical cutting device according to one or more of the preceding claims, **characterised in that** the loop arc (7) is formed of a wire cable.

5. Surgical cutting device according to one or more of the preceding claims, **characterised in that** the loop arc (7), in the region of the peak thereof, forms a bight (11) having loop arc portions (11') which extend at an acute angle or parallel to each other.

6. Surgical cutting device according to one or more of the preceding claims, **characterised in that** the traction elements (10) which engage with the free ends (6') of the expansion legs (6) are formed of wire cables.

7. Surgical cutting device according to one or more of the preceding claims, **characterised in that** the stop (8) is formed of a length of tubing which is slidably mounted in the sleeve (1), through which length of tubing the expansion legs (6) protrude.

8. Surgical cutting element according to one or more of the preceding claims, **characterised in that** the traction elements (10) extend along the wide side faces, which face away from each other, of the expansion legs (6).

9. Surgical cutting device according to one or more of the preceding claims, **characterised in that** the free ends (6') of the expansion legs are located in the direction of the apex of the arc of the loop (4) in front of the region of the greatest opening width of the loop (4).

10. Surgical cutting device according to one or more of the preceding claims, **characterised in that** at least the loop arc (7) comprises a plurality of local thickenings (12) which are spaced apart from each other.

11. Surgical cutting device according to one or more of the preceding claims, **characterised in that** at least the loop arc (7) comprises a plurality of hooks (13) which are spaced apart from each other and directed towards the inside of the loop.

12. Surgical cutting device according to one or more of the preceding claims, **characterised in that** the loop (4) consists of an electrically conductive material and is electrically conductively connected to an electrical contact (14) of the actuation means via the traction/thrust element (2) formed as a band or cable.

## Revendications

1. Appareil de coupe chirurgical comprenant un élément de traction / de poussée (2) guidé dans une douille (1), destiné à transmettre une force de traction / de poussée d'un dispositif d'actionnement (3) disposé à une des extrémités de la douille (1) sur une boucle (4) disposée à l'autre extrémité de la douille (1) pour déplacer la boucle (4) d'une position de stockage, dans laquelle la boucle (4) se trouve dans un état d'extension dans la douille (1), dans une position d'utilisation, dans laquelle l'ouverture (5) déployée de la boucle (4) se trouve devant la douille (1), et inversement, la boucle formant deux branches de déploiement (6) déployées en forme de V qui se trouvent en partie dans et devant la douille (1) dans la position d'utilisation et dont les extrémités libres (6') sont reliées par un arc (7) de boucle, **caractérisé en ce que** une butée (8) déplaçable avec la boucle (4), placée de manière coulissante dans la douille (1), bute contre une contre-butée (9) fixée à la douille lors du déplacement de la boucle (4) de sa position de stockage dans sa position d'utilisation pour exercer une action de déploiement sur la boucle (4) et **en ce que** des éléments de traction (10) supplémentaires, qui s'étendent de manière pratiquement parallèle aux branches de déploiement (6) et sont reliés à la butée mobile (8) par rapport aux branches de déploiement (6), s'appliquent à chacune des extrémités libres (6').

2. Appareil de coupe chirurgical selon la revendication 1, **caractérisé en ce que** les branches de déploiement (6) déployées en forme de V sont plus rigides à la flexion que l'arc (7) de boucle au moins dans la direction transversale au plan d'ouverture (5).

3. Appareil de coupe chirurgical selon une ou plusieurs des revendications précédentes, **caractérisé en ce que** les branches de déploiement (6) sont formées par des bandes plates à déformation élastique qui reposent l'une contre l'autre par leurs surfaces du côté large dans la position de stockage.

4. Appareil de coupe chirurgical selon une ou plusieurs des revendications précédentes, **caractérisé en ce que** l'arc (7) de boucle est formé par un câble métallique.

5. Appareil de coupe chirurgical selon une ou plusieurs des revendications précédentes, **caractérisé en ce que** l'arc (7) de boucle forme une anse (11) dans la région de sa pointe, les parties (11') d'arc de boucle s'étendant à un angle aigu ou parallèlement l'une à l'autre.

6. Appareil de coupe chirurgical selon une ou plusieurs des revendications précédentes, **caractérisé en ce que** les éléments de traction (10) s'appliquant aux extrémités libres (6') des branches de déploiement (6) sont formés par des câbles métalliques.

7. Appareil de coupe chirurgical selon une ou plusieurs des revendications précédentes, **caractérisé en ce que** la butée (8) est formée par une pièce tubulaire montée de manière coulissante dans la douille (1) et à travers laquelle les branches de déploiement (6) font saillie.

8. Appareil de coupe chirurgical selon une ou plusieurs des revendications précédentes, **caractérisé en ce que** les éléments de traction (10) s'étendent le long des surfaces du côté large orientées en sens opposé des branches de déploiement (6).

9. Appareil de coupe chirurgical selon une ou plusieurs des revendications précédentes, **caractérisé en ce que** les extrémités libres (6') des branches de déploiement (6) se trouvent avant la région de la plus grande largeur d'ouverture de la boucle (4) en direction du sommet d'arc de la boucle (4).

10. Appareil de coupe chirurgical selon une ou plusieurs des revendications précédentes, **caractérisé en ce qu'**au moins l'arc (7) de boucle présente une pluralité d'épaississements (12) locaux espacés les uns des autres.

11. Appareil de coupe chirurgical selon une ou plusieurs des revendications précédentes, **caractérisé en ce qu'**au moins l'arc (7) de boucle présente une pluralité de crochets (13) espacés les uns des autres, orientés vers l'intérieur de la boucle.

12. Appareil de coupe chirurgical selon une ou plusieurs des revendications précédentes, **caractérisé en ce que** la boucle (4) est constituée d'un matériau électroconducteur et est reliée de manière électroconductrice au contact électrique (14) du dispositif d'actionnement par l'intermédiaire de l'élément de traction / de poussée (2) réalisé comme bande ou câble.
